# EUROPÄISCHE PATENTSCHRIFT

(11) **EP 1 507 783 B1**
(45) Veröffentlichungstag und Bekanntmachung des Hinweises auf die Patenterteilung: **27.12.2006**
(21) Anmeldenummer: 03732426.6
(22) Anmeldetag: 20.05.2003
(51) Int. Cl.: C07F 9/50, C07F 15/00, C07B 53/00, C07C 45/00

(54) **HYDROXYDIPHOSPHINE UND DEREN VERWENDUNG IN DER KATALYSE**
HYDROXY DIPHOSPHINES AND THEIR USE IN CATALYSIS
HYDROXYDIPHOSPHINES ET LEUR UTILISATION EN CATALYSE

(30) Priorität: 27.05.2002 DE 10223593
(43) Veröffentlichungstag der Anmeldung: 23.02.2005
(73) Patentinhaber: Degussa AG, 40474 Düsseldorf (DE)
(72) Erfinder: KOMAROV, Igor, 03115 Kiev (UA); BÖRNER, Armin, 18055 Rostock (DE); MONSEES, Axel, 60487 Frankfurt (DE); KADYROV, Renat, 65931 Frankfurt (DE)
(86) Internationale Anmeldenummer: PCT/EP2003/005286
(87) Internationale Veröffentlichungsnummer: WO 2003/099832

(56) Entgegenhaltungen:
- KOMAROV I V ET AL: "A new hydroxydiphosphine as a ligand for Rh(I)-catalyzed enantioselective hydrogenation" TETRAHEDRON: ASYMMETRY, ELSEVIER SCIENCE PUBLISHERS, AMSTERDAM, NL, Bd. 13, Nr. 15, 14. August 2002 (2002-08-14), Seiten 1615-1620, XP004385886 ISSN: 0957-4166
- BÖRNER A.: "The effect of internal hydroxy groups in chiral diphosphane Rhodium(I) catalysts on the asymmetric hydrogenation of functionalized olefins" EUROPEAN JOURNAL OF INORGANIC CHEMISTRY., Nr. 2, 2001, Seiten 327-337, XP002256141 WILEY-VCH VERLAG, WEINHEIM., DE ISSN: 1434-1948 in der Anmeldung erwähnt

## Beschreibung

Die vorliegende Erfindung beinhaltet neue unsymmetrische chirale Hydroxydiphosphine sowie deren Derivate, deren Synthese sowie Komplexe dieser Verbindungen mit Übergangsmetallen und deren Verwendung als Katalysatoren für enantioselektive Transformationen, insbesondere für Hydrierungen.

Bidentate Organophosphorverbindungen haben große Bedeutung als Liganden in der homogenen Katalyse erlangt. Neben den reinen bidentaten Organophosphorphorverbindungen haben sich Verbindungen mit einer weiteren Koordinationsstelle als interessante Komplex-Liganden, insbesondere bei der Entwicklung neuer Katalysatoren, besonders in asymmetrischen Synthesen, erwiesen (Börner, A.; Eur. J. Inorg. Chem. 2001, 327-337). Ein wichtiger Aspekt des Erfolges der Diphosphinliganden wird der Schaffung einer besonders asymmetrischen Umgebung des Metallzentrums zugeschrieben, wobei allerdings viele dieser Liganden bei katalytischen Umsetzungen noch nicht ausreichende Ausbeuten liefern. Ein Ansatz dieses Problem zu beheben liegt in der Verwendung von modifizierten Diphosphinliganden.

Es besteht daher ein großes Interesse an chiralen Organophosphor-Liganden die eine weitere Koordinationsstelle enthalten und die darüber hinaus einfach in einer Vielzahl von strukturellen Varianten herstellbar sind, die sich stereochemisch und elektronisch unterscheiden, um den für eine bestimmte asymmetrische Katalyse optimalen "maßgeschneiderten" Liganden auffinden zu können.

Der vorliegenden Erfindung liegt die Aufgabe zugrunde neuartige, unsymmetrische, bidentate und chirale Phosphorligandsystemen, die eine zusätzliche Koordinationsstelle für das Substrat besitzen, bereitzustellen.

Die Aufgabe wird durch eine Klasse chiraler, unsymmetrischer bidentater Organophosphorverbindungen der Formel (I) gelöst.

Die vorliegende Erfindung betrifft somit Verbindungen der allgemeinen Formel (I), 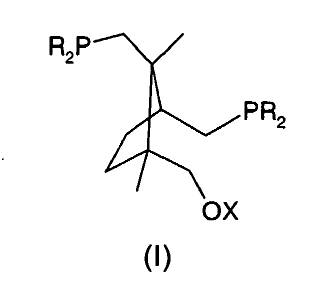 worin das Kohlenstoffgerüst der Verbindungen der Formel (I), neben den bevorzugten Wasserstoffsubstituenten, ein oder mehrere lineare, verzweigte oder cyclische (C₁-C₂₀)-Alkylsubstituenten tragen kann und worin die einzelnen Reste
- R: unabhängig voneinander für einen Rest ausgewählt aus der Gruppe (C₁-C₂₄)-Alkyl, (C₃-C₈)-Cycloalkyl, (C₆-C₁₄)-Aryl, Phenyl, Naphthyl, Fluorenyl, (C₂-C₁₃)-Heteroaryl, wobei die Zahl der Heteroatome, ausgewählt aus der Gruppe N, O, S, 1-2 betragen kann, stehen.
Die vorgenannten Reste selbst können jeweils ein- oder mehrfach substituiert sein. Diese Substituenten können dabei unabhängig voneinander Wasserstoff, (C₁-C₂₀)-Alkyl, (C₂-C₂₀)-Alkenyl, (C₁-C₁₀)-Haloalkyl, (C₃-C₈)-Cycloalkyl, (C₂-C₉)-Heteroalkyl, (C₆-C₁₀)-Aryl, Phenyl, Naphthyl, Fluorenyl, (C₂-C₆)-Heteroaryl, wobei die Zahl der Heteroatome, insbesondere aus der Gruppe N, O, S, 1-4 betragen kann, (C₁-C₁₀)-Alkoxy, bevorzugt OMe, (C₁-C₉)-Trihalomethylalkyl, bevorzugt Trifluormethyl und Trichlormethyl, Halogeno, besonders Fluoro und Chloro, Hydroxy, substituierte Amino der Formen NH-Alkyl-(C₁-C₈), NH-Aryl-(C₅-C₆), N(Alkyl-(C₁-C₈))₂, N(Aryl-(C₅-C₆))₂, N-Alkyl-(C₁-C₈)⁺₃, N-Aryl-(C₅-C₆)⁺₃, NH-CO-Alkyl-(C₁-C₈), NH-CO-Aryl-(C₅-C₆), Carboxylato der Formen COOH und COOQ wobei Q entweder ein einwertiges Kation oder (C₁-C₈)-Alkyl darstellt, (C₁-C₆)-Acyloxy, Sulfinato, Sulfonato der Formen SO₃H und SO₃Q, wobei Q entweder ein einwertiges Kation, (C₁-C₈)-Alkyl oder C₆-Aryl darstellt, Tri-(C₁-C₆)-Alkylsilyl, besonders SiMe₃ sind,
und/oder wobei zwei Reste R miteinander verbrückt sein können, wobei bevorzugt ein 4-8 gliedriger Zyklus gebildet wird, der gegebenenfalls mit linearen oder verzweigten Resten ausgewählt aus der Gruppe (C₁-C₁₀)-Alkyl, C₆-Aryl, Benzyl, (C₁-C₁₀)-Alkoxy, Hydroxy und Benzyloxy, substituiert ist.
- X: kann Wasserstoff, linearen oder verzweigten (C₁-C₁₀)-Alkyl, C₆-Aryl, C(O)Y sein, wobei Y für einen linearen oder verzweigten (C₁-C₁₀)-Alkyl-, C₆-Aryl- oder Benzylrest stehen kann.
- P: ist ein dreiwertiger Phosphor.

Die Erfindung betrifft ferner Komplexverbindungen, die einen chiralen bidentaten Organophosphorliganden der Formel (I) mit mindestens einem Metall enthalten. Solche Komplexverbindungen sind durch einfaches Zusammengeben der erfindungsgemäßen Organophosphorverbindungen mit Metallkomplexprecursoren in Lösung erhältlich.

Aus der Gruppe der Alkylsubstituenten sind bevorzugt Methyl, Ethyl, n-Propyl, 1-Methylethyl, n-Butyl, 1-Methylpropyl, 1,1-Dimethylethyl, n-Pentyl, 1-Methylbutyl, 2-Methylbutyl, 3-Methylbutyl, 2,2-Dimethylpropyl, 1-Ethylpropyl, n-Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, n-Heptyl, n-Octyl, n-Nonyl, n-Decyl.

Unter den cyclischen Alkylsubstituenten sind besonders bevorzugt substituierte und unsubstituierte Cyclopentyl-, Cyclohexyl-, Cycloheptylreste.

Unter den Arylsubstituenten sind besonders bevorzugt 2-Alkylphenyl, 3-Alkylphenyl, 4-Alkylphenyl, 2,6-Dialkylphenyl, 3,5-Dialkylphenyl, 3,4,5-Trialkylphenyl, 2-Alkoxyphenyl, 3-Alkoxyphenyl, 4-Alkoxyphenyl, 3,5-Dialkoxyphenyl, 3,5-Dialkyl-4-Alkoxyphenyl, 3,5-Trifluormethylphenyl, 4-Trifluormethylphenyl

Schließlich sind Ligandsysteme der Formel (I) als optisch aktive Ligandsysteme bevorzugt, bei denen ein Enantiomer angereichert ist. Besonders bevorzugt sind Ligandsysteme, bei denen die Enantiomerenanreicherung 90 %, insbesondere 99 % übersteigt.
Organophosphorverbindungen der Formel (I) sind in der Lage in metallorganischen Komplexen am Metallzentrum eine hochasymmetrische Koordinationssphäre mit unabhängig voneinander modifizierbaren Organophosphordonoren zu schaffen und somit eine effektive asymmetrische Induktion ermöglichen. Zusätzlich können über die einfache Einführbarkeit unterschiedlichster Substituenten in die Organophosphorliganden die Flexibilität der Koordinationssphäre des Komplexes sterisch kontolliert werden.

Damit kann für Verbindungen der Formel (1) ein breites Spektrum von Anwendungen erschlossen werden, da die zweizähnigen Phosphorligandenliganden sterisch und elektronisch, je nach katalystischem Syntheseverfahren durch die Einführung geeigneter Substituenten optimierbar sind.

Erfindungsgemäße Phosphorverbindungen aus der Klasse der Hydroxyphosphine können z. B. durch das im folgenden beschriebenen neue Verfahren hergestellt werden.

Die Synthese nutzt "chiral pool" Verbindungen wie z. B. (R)-Campher. Anhand eines von Money (*Nat. Prod. Rep.* **1985**, *2*, 253) und von Lawrence *(Ph.D. Thesis, UCLA,* **1982**) beschriebenen Verfahren läßt sich die 9-Brom-Campher-Verbindung in einer dreistufigen Synthese herstellen. Die Synthese kann ebenfalls von käuflich erhältlichen 3-Brom-Campher begonnen werden.

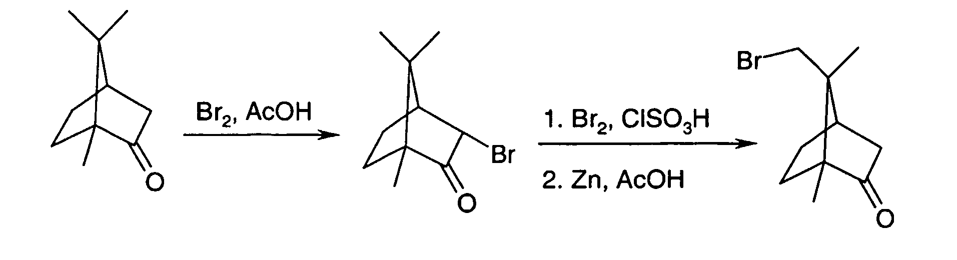

Das (2-Brommethyl-3-hydroxymethyl-2,3-dimethylcyclopentyl)-methanol wird durch stufenweise Oxidation von 9-Brom-Campher mit Selendioxid und Wasserstoffperoxid, gefolgt durch eine Reduktion erhalten. Als besonders vorteilhaft hat sich eine Bayer-Villinger Reaktion mit nachfolgender Reduktion erwiesen.

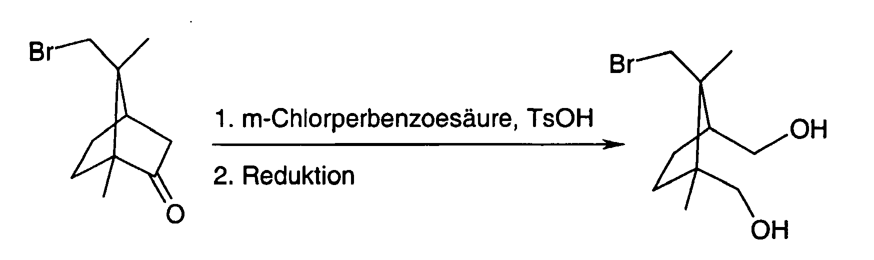

Überraschender Weise konnte gezeigt werden, dass das Diol ausgehend von (S)-Campher ebenfalls eine sehr hohe optische Reinheit (98%ee) zeigt, obwohl das Ausgangsmaterial nur optische Reinheiten von 79-93%ee besitzt.

Das Diol wird an der sterisch weniger gehinderten Position selektiv in das Mono-Tosylat überführt. In einem weiteren Reaktionschritt wird die freie Hydroxygruppe durch eine geeignete Schutzgruppe blockiert. Anstelle der Tosylgruppe kann an dieser Stelle auch eine andere funktionelle Gruppe stehen, die die nucleophile Substitution im weiteren Reaktionsverlauf erleichtert.

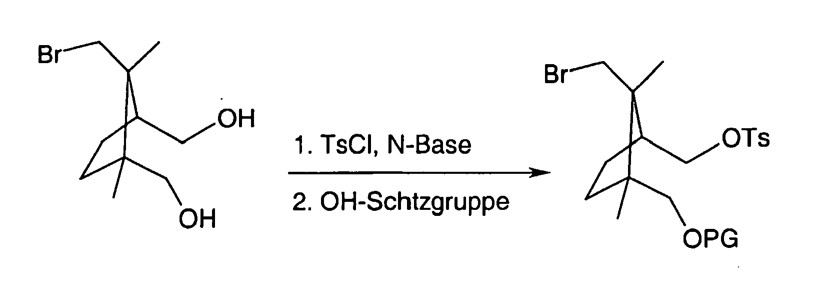

Durch eine nucleophile Substitution mit einem phosphorhaltigen Reagenz, das kommerziell zur Verfügung steht, bzw. mit für diesen Zweck geläufigen Reagenzien vom Fachmann hergestellt werden kann (siehe dazu z. B. A Guide To Organophophorus Chemistry, Louis D. Quin, John Wiley & Sons 2000), wird das Diphosphin gebildet. Eine nachfolgende Abspaltung der Schutzgruppe liefert die erfindungsgemäßen Liganden, der sich durch seine C1-Symmetrie und einer zusätzlichen freien Koordinationstelle auszeichnet.

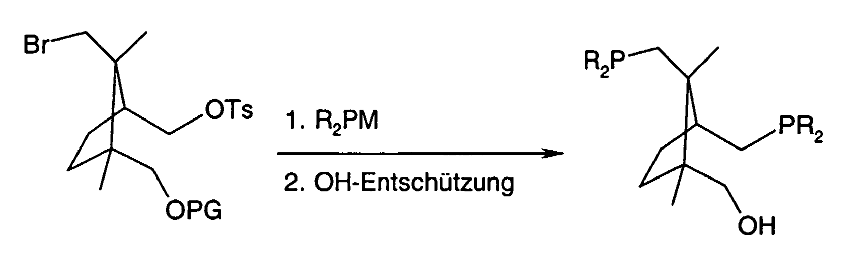

Das Ligandensystem kann mit dem Fachmann geläufigen Methoden im Rahmen des Anspruchsumfangs beliebig abgewandelt werden, insbesondere durch weitere Variationen an der freien Hydroxygruppe, z. B. durch deren Veresterung oder Veretherung.

Die erfindungsgemäßen Verbindungen sind mit dem beschriebenen Herstellungsverfahren, im Gegensatz zu vielen etablierten Ligandsystemen, besonders einfach in großer Variationsbreite, ausgehend von einfachen Edukten, zugänglich. Somit können durch Variation der Substituenten am Phosphor und Sauerstoff die elektronischen und sterischen Eigenschaften des erfindungsgemäßen Liganden gezielt beeinflussen werden, so dass Ausbeute, Selektivität und Aktivität bei homogen-katalytischen Prozessen gesteuert werden können.

Durch die leichte Zugänglichkeit können die Liganden für die industrielle Herstellung erschlossen werden.

Die Verbindungen der allgemeinen Formel (1) können als Liganden an Übergangsmetallen in asymmetrischen, Metall-katalysierten Reaktionen, wie z.B. der Hydrierung, der Hydroformylierung, der Umlagerung, der allylischen Alkylierung, der Cyclopropanierung, der Hydrosilylierung, den Hydridübertragungen, Hydroborierungen, Hydrocyanierungen, Hydrocarboxylierungen, Aldol-Reaktionen oder der Heck-Reaktion, sowie bei Polymerisationen eingesetzt werden. Sie sind insbesondere für asymmetrische Reaktionen gut geeignet.

Die erfindungsgemäßen Liganden sind besonders geeignet in der asymmetrischen Hydrierung von C=C-, C=O- oder C=N-Bindungen, in denen sie hohe Aktivitäten, Ausbeuten, teilweise bis zu 100%, und Selektivitäten aufweisen, sowie in der asymmetrischen Hydroformylierung.

Als besonders geeignete Katalysatoren für Hydrierungen haben sich z. B. Ru- und Rh-Komplexe enthaltend einen erfindungsgemäßen Liganden der Formel (I) erwiesen.

Die katalysatischen Komplexverbindungen können entweder direkt in einer Ein-Topf-Reaktion durch einfaches zusammengeben von erfindungsgemäßem Ligand und Metall, Metallsalz oder Metallvorkomplex gebildet werden oder auch vorab hergestellt und isoliert und dem Reaktionsansatz als fertige Komplexverbindung zugegeben werden.

Geeignete Katalysatoren stellen z. B. Komplexverbindungen der allgemeinen Formel (II) dar, die erfindungsgemäße Verbindungen der Formel (I) als Liganden enthalten,

[MₓP_{y}L_{z}S_{q}]Aᵣ (II)

wobei in der allgemeinen Formel (II) M ein Übergangsmetallzentrum, bevorzugt der Gruppen Vllb, Vlllb und Ib des Periodensystems, L gleiche oder verschiedene koordinierende organische oder anorganische Liganden und P erfindungsgemäße bidentate Organophosphorliganden der Formel (I) darstellen, S koordinierende Lösungsmittelmoleküle und A Äquivalente aus nicht koordinierenden Anionen repräsentiert, wobei x 1 oder 2 ist, y eine ganze Zahl größer oder gleich 1 ist und z, q und r unabhängig voneinander ganze Zahlen größer oder gleich 0 sind. Die Summe y + z + q wird durch die an den Metallzentren zur Verfügung stehenden Koordinationszentren nach oben begrenzt, wobei nicht alle Koordinationsstellen besetzt sein müssen.

Die erfindungsgemäßen Komplexverbindungen enthalten mindestens ein Übergangsmetallatom oder -ion, insbesondere aus der Gruppe Palladium, Platin, Rhodium, Ruthenium, Osmium, Iridium, Kobalt, Nickel, oder/und Kupfer.

Bevorzugte Liganden L solcher Komplexverbindungen sind Halogenid, besonders Cl, Br und 1, Dien, besonders Cyclooctadien, Norbornadien, Olefin, besonders Ethylen und Cycloocten, Acetato, Trifluoracetato, Acetylacetonato, Allyl, Methallyl, Alkyl, besonders Methyl und Ethyl, Nitril, besonders Acetonitril und Benzonitril, sowie Carbonyl und Hydrido Liganden.

Bevorzugte koordinierende Lösungsmittel S sind Amine, besonders Triethylamin, Alkohole, besonders Methanol und Aromaten, besonders Benzol und Cumol.

Bevorzugte nichtkoordinierende Anionen A sind Trifluoracetat, Trifluormethansulfonat, BF₄; ClO₄, PF₆, SbF₆, und BAr₄.

In den einzelnen Komplexverbindungen können dabei unterschiedliche Moleküle, Atome oder Ionen der einzelnen Bestandteile M, P, L, S und A enthalten sein.

Die Herstellung dieser Metall-Ligand-Komplexverbindungen kann in situ durch Reaktion eines Metallsalzes oder eines entsprechenden Vorkomplexes mit den Liganden der allgemeinen Formel (I) erfolgen. Darüber hinaus kann eine Metall-Ligand-Komplexverbindung durch Reaktion eines Metallsalzes oder eines entsprechenden Vorkomplexes mit den Liganden der allgemeinen Formel (I) und anschließende Isolierung gewonnen werden. Die Erzeugung einer solchen Komplexverbindung erfolgt bevorzugt in einer Eintopfreaktion unter Rühren bei erhöhter Temperatur. Katalytisch aktive Komplexverbindungen können dabei auch direkt im Reaktionsansatz der geplanten katalytischen Umsetzung erzeugt werden.

Beispiele für die Metallsalze sind Metallchloride, -bromide, -iodide, -cyanide, -nitrate, -acetate, -acetylacetonate, -hexafluoracetylacetonate, tetrafluoroborate, - perfluoracetate oder -triflate, insbesondere des Palladium, Platins, Rhodium, Ruthenium, Osmium, Iridium, Kobalts, Nickels oder/und des Kupfers. Beispiele für die Vorkomplexe sind:
Cyclooctadienpalladiumchlorid, Cyclooctadienpalladiumiodid,
1,5-Hexadienpalladiumchlorid, 1,5-Hexadienpalladiumiodid,
Bis(dibenzylidenaceton)palladium, Bis(acetonitril)palladium(II)chlorid,
Bis(acetonitril)palladium(II)bromid, Bis(benzonitril)palladium(II)chlorid,
Bis(benzonitril)palladium(II)bromid, Bis(benzonitril)palladium(II)iodid,
Bis(allyl)palladium, Bis(methallyl)palladium, Allylpalladiumchlorid-Dimer,
Methallylpalladiumchlorid-Dimer, Tetramethylethylendiaminpalladiumdichlorid,
Tetramethylethylendiaminpalladiumdibromid,
Tetramethylethylendiaminpalladiumdiiodid,
Tetramethylethylendiaminpalladiumdimethyl,
Cyclooctadienplatinchlorid, Cyclooctadienplatiniodid, 1,5-Hexadienplatinchlorid,
1,5-Hexadienplatiniodid, Bis(cyclooctadien)platin, Kalium(ethylentrichloroplatinat),
Cyclooctadienrhodium (I) chlorid-Dimer, Norbornadienrhodium (I) chlorid-Dimer,
1,5-Hexadienrhodium(I)chlorid-Dimer, Tris(triphenylphosphan)rhodium(I)chlorid,
Hydridocarbonyltris(triphenylphosphan)rhodium(I)chlorid,
Bis(cyclooctadien)rhodium(I)perchlorat, Bis(cyclooctadien)rhodium(I)tetrafluorborat,
Bis(cyclooctadien)rhodium(I)triflat,
Bis(acetonitril)(cyclooctadien)rhodium(I)perchlorat,
Bis(acetonitril)(cyclooctadien)rhodium(I)tetrafluorborat,
Bis(acetonitril)(cyclooctadien)rhodium(I)triflat,
Cyclopentadienrhodium(III)chlorid-Dimer,
Pentamethylcyclopentadienrhodium(III)chlorid-Dimer,
(cyclooctadien)Ru(η³-allyl)₂, ((cyclooctadien)Ru)₂(acetat)₄,
((Cyclooctadien)Ru)₂(trifluoracetat)₄, RuCl₂(Aren)-Dimer,
Tris(triphenylphosphan)ruthenium(II)chlorid, Cyclooctadienruthenium(II)chlorid,
OsCl₂(Aren)-Dimer, Cyclooctadieniridium(I)chlorid-Dimer,
Bis(cycloocten)iridium(I)chlorid-Dimer,
Bis(cyclooctadien)nickel, (Cyclododecatrien)nickel, Tris(norbornen)nickel,
Nickeltetracarbonyl, Nickel(II)acetylacetonat,
(Aren)kupfertriflat, (Aren)kupferperchlorat, (Aren)kupfertrifluoracetat, Kobaltcarbonyl.

### Ausführungsbeispiele :

Beispiel 1: (1 R,5R,8R)-8-(Brommethyl)-1,8-dimethyl-3-oxabicyclo[3.2.1]octan-2-on Eine Lösung von 4.3 mmol (1.0 g) (+)-9-Bromcampher, 6.5 mmol (1.12 g) m-Chlorperbenzoesäure und 100 mg p-Toluolsulfonsäure in 40 ml Dichlormethan wird für 4 Tage unter Rückfluß erhitzt. Täglich werden weitere 40 mg m-Chlorperbenzoesäure in die Reaktionslösung gegeben. Nach vier Tagen wird die Lösung mit 100 ml Ether verdünnt, mit ges. Natriumhydrogencarbonat-Lösung, mit Natriumsulfit-Lösung, erneut mit ges. Natriumhydrogencarbonat-Lösung und mit Wasser gewaschen. Die organische Phase wird getrocknet und das Lösungsmittel im Vakuum entfernt. Das Rohprodukt wird mittels Chromatographie gereinigt und das Produkt mit einer Ausbeute von 33% erhalten.
¹H-NMR (CDCl₃):
δ = 4.41 (ddd, 1H), 4.15 (d, 1H), 3.39 (dq, 1 H), 3.25 (d, 1 H), 2.46 (dd, 1 H), 2.08 (m, 2H), 1.95 (m, 2H), 1.18 (d, 3H), 1.16 (s, 3H) ppm.

### Beispiel 2: (1 R,2R,3S)-(2-Brommethyl-3-hydroxymethyl-2,3-dimethylcyclopentyl)-methanol

Es werden 0.61 mmol (150 mg) (1 R,5R,8R)-8-(Brommethyl)-1,8-dimethyl-3-oxabicyclo[3.2.1]octan-2-on in 10 ml Ether gelöst. Zu dieser Lösung wird in kleinen Portionen 1.22 mmol (46.1 mg) Lithiumaluminiumhydrid gegeben und die Reaktionslösung weitere 15 min gerührt. Die Reaktionlösung wird mit Wasser hydrolysiert, der Rückstand abfiltriert und mehrmal mit Ether gewaschen. Das Filtrat wird getrocknet und das Lösungsmittel entfernt. Das Rohprodukt wird in quantitativer Ausbeute erhalten und ohne weitere Reinigung eingesetzt.
¹H-NMR (CD₃OD):
δ = 3.48-3.65 (m, 4H), 3.22-3.33 (m, 4H), 2.08 (m, 1 H), 1.75-1.86 (m, 1 H), 1.60-1.70 (m, 1H), 1.26-1.38 (m, 2H), 0.97 (s, 3H), 0.88 (s, 3H) ppm.

### Beispiel 3: [(1R,2R,3S)-(2-Brommethyl)-3-hydroxymethyl)-2,3-dimethylcyclopentyl]-methyl-4-tosylat

Es werden 10.7 mmol (2.05 g) p-Toluolsulfonsäurechlorid innerhalb von 15 min bei - 10°C zu einer Lösung von 10.7 mmol (2.7 g) (1 R,2R,3S)-(2-Brommethyl-3-hydroxymethyl-2,3-dimethylcyclopentyl)-methanol in 10 ml Pyridin gegeben. Die Lösung wird für 45 min bei dieser Temperatur gerührt und anschließend mit 75 ml Wasser hydrolysiert. Die wäßrige Phase wird mit 150 ml Ether extrahiert. Anschließend wird die organische Phase mit 50 ml Wasser, 5%iger Salzsäure und Wasser gewaschen. Nach dem Trocknen der organischen Phase wird das Lösungsmittel im Vakuum entfernt und das Rohprodukt mittels Chromatographie gereinigt. Das Produkt wird sofort in die nächste Reaktionsstufe eingesetzt.
¹H-NMR (CDCl₃):
δ = 7.77 (d, 2H), 7.34 (d, 2H), 4.30 (d, 1 H), 4.01 (d, 1 H), 3.75 (dd, 1 H), 3.67 (d, 1 H), 3.58 (dd, 1H), 3.54 (d, 1 H), 2.44 (s, 3H), 2.15 (m, 1 H), 1.85 (m, 2H), 1.62 (m, 1 H), 1.48 (m, 1 H), 1.29 (m, 1 H), 1.00 (s, 3H), 0.93 (s, 3H) ppm.

### Beispiel 4: [(1 R,2R,3S)-(2-Brommethyl)-2,3-dimethyl-3-[(tetrahydro-2H-pyran-2-yloxy)methyl]cyclopentyl]-methyl-4-methylbenzolsulfonat

7.3 mmol (2.96 g) [(1R,2R,3S)-(2-Brommethyl)-3-hydroxymethyl)-2,3-dimethylcyclopentyl]-methyl-4-tosylat werden in 20 ml Dichlormethan gelöst. Zu dieser Lösung werden 10.95 mmol (1 ml) 3,4-Dihydro-2H-pyran und Pyridinium-p-Toluolsulfonat gegeben und über Nacht gerührt. Die Reaktionslösung wird anschließend mit 100ml Ether verdünnt, mit Natriumchlorid-Lösung und Wasser gewaschen. Die organische Phase wird getrocknet und das Lösungsmittel im Vakuum entfernt. Das Rohprodukt wird in quantitativer Ausbeute erhalten und ohne weitere Reinigung eingesetzt.
¹H-NMR (CDCl₃):
δ =7.76 (d, 2H), 7.33 (d, 2H), 4.50 (m, 2H), 4.28 (dd, 1 H), 3.00-4.00 (m, 6H), 2.44 (s, 3H), 1.10-2.40 (m, 11 H), 0.85-1.05 (m, 6H) ppm.

### Beispiel 5: [(1R,2R,3S)-1,2-Dimethyl-2,3-bis(diphenylphosphinomethyl)cyclopentyl]methanol

Zu einer Lösung von 2.3 mmol Lithiumdiphenylphosphid in 10 ml THF wird unter Eiskühlung eine Lösung von 0.94 mmol (0.46 g) [(1 R,2R,3S)-(2-Brommethyl)-2,3-dimethyl-3-[(tetrahydro-2H-pyran-2-yloxy)methyl]cyclopentyl]-methyl-4-methylbenzolsulfonat gelöst in 5 ml THF zugegeben. Die Lösung wird 2h bei Raumtemperatur gerührt und anschließend 90 min refluxiert. Die Reaktionlösung wird mit 20 ml Wasser hydrolysiert und die wäßrige Phase mit 70 ml Ether extrahiert. Anschließend wird die organische Phase zweimal mit Wasser gewaschen und 4h im Vakuum bei 50°C getrocknet. Der Rückstand wird in 8 ml Ethanol aufgenommen und es werden 25 mg Pyridinium-p-Toluolsulfonnat zugegeben. Die Lösung wird 2 Tage bei 55°C gerührt. Das Lösungsmittel wird im Vakuum entfernt und das Rohprodukt mittels Chromatographie gereinigt. Die Verbindung fällt in 43% Ausbeute als Öl an.
¹H-NMR (C₆D₆):
δ = 7.75 (t, 2H), 7.58-7.62 (m, 4H), 7.57 (t, 2H), 6.95-7.10 (m, 12H), 3.75 (d, 1 H), 3.30 (d, 1 H), 3.48 (br, s, 1 H), 3.12 (dq, 1 H), 1.90-2.00 (m, 1 H), 2.63 (dd, 1 H), 2.32 (dd, 1 H), 2.19-2.30 (dd, 1 H), 2.19-2.30 (m, 2H), 1.15-1.50 (m, 3H), 1.02 (s, 3H), 0.92 (s, 3H) ppm.
³¹P-NMR (C₆D₆):
δ = -15.7, -22.7 ppm.

### Beispiel 6: Herstellung der Rh-Komplexes

Zu einer Lösung von 1 mmol des Diphosphines (Beispiel 5) in 2 ml THF werden 1 mmol [Rh(COD)acac] gegeben und die Lösung 15 min gerührt. Im Anschluß wird eine äquimolare Menge 40%ige Tetrafluorboronsäure zugegeben und weitere 15 min gerührt. Der Metallkomplex wird durch Zugabe von 20 ml Ether ausgefällt, durch Zugabe von 0.5 ml Dichlormethan wieder gelöst und durch Zugabe von Ether erneut ausgefällt. Der Metallkomplex wird abfiltriert und im Vakuum getrocknet.
³¹P-NMR (CDCl₃):
δ = 22.3 (dd), 16.7 (dd) ppm.

### Beispiel 7: Hydrierungen

Alle Hydrierungen wurden bei 25°C unter einem Wasserstoffdruck von 1 bar in 15 ml Lösungsmittel durchgeführt. Substrat und Katalysator (Beispiel 6) wurden im Verhältnis 100:1 eingesetzt.

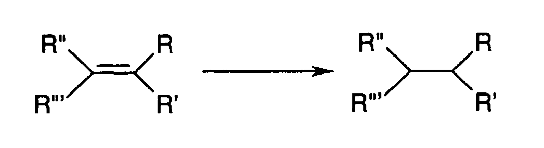

| R | R' | R" | R"' | Lösungsmittel | Zeit | Umsatz % | ee % |
|---|---|---|---|---|---|---|---|
| COOMe | NHAc | H | Ph | MeOH | 1.5 min | 100 | 11 (S) |
| COOMe | NHAc | H | Ph | CH₂Cl₂ | 5 min | 100 | 24 (S) |
| COOH | NHAc | H | H | MeOH | 1.5 min | 100 | 57 (S) |
| COOH | NHAc | H | H | CH₂Cl₂ | 8 min | 100 | 49 (S) |
| CH₂COOMe | COOMe | H | H | MeOH | 6 min | 100 | 70 (S) |
| CH₂COOMe | COOMe | H | H | CH₂Cl₂ | 3.5 min | 100 | 75 (S) |
| CH₂COOH | COOH | H | H | MeOH | 1.5 min | 100 | 9 (S) |
| CH₂COOH | COOH | H | H | CH₂Cl₂ | 300 min | 62 | 10 (S) |
| COOMe | H | Me | NHAc | MeOH | 40 min | 100 | 93 (R) |
| COOMe | H | Me | NHAc | CH₂Cl₂ | 12 min | 100 | 95 (R) |
| COOMe | H | Me | NHAc | Toluol | 300 min | 98 | 97 (R) |
| COOMe | H | NHAc | Me | MeOH | 20 min | 100 | 46 (R) |
| COOMe | H | NHAc | Me | CH₂Cl₂ | 45 min | 72 | 58 (R) |
| COOMe | H | NHAc | Me | Toluol | 700 min | 7 | 50 (R) |
| COOH | H | Me | NHAc | MeOH | 120 min | 82 | 81 (R) |
| COOH | H | Me | NHAc | CH₂Cl₂ | 24h | 54 | 82 (R) |
| COOH | H | NHAc | Me | MeOH | 13h | 53 | 54 (R) |
| COOH | H | NHAc | Me | CH₂Cl₂ | 24h | 8 | 12 (R) |
| CONH(CH₂)₂ COOMe | H | Me | NHAc | CH₂Cl₂ | 13h | 50 | 80 |
| CONH(CH₂)₂ COOMe | H | Me | NHAc | CH₂Cl₂ | 13h | 90 | 33 |

## Patentansprüche

1. Diphosphine der Formel (I), 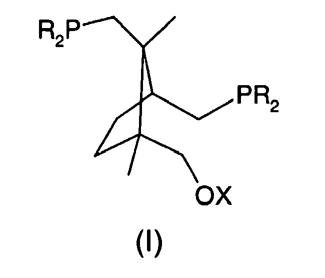 worin das Kohlenstoffgerüst der Verbindungen der Formel (I) ein oder mehrere lineare, verzweigte oder cyclische (C₁-C₂₀)-Alkylsubstituenten tragen kann und worin die einzelnen Reste
R unabhängig voneinander für einen Rest ausgewählt aus der Gruppe (C₁-C₂₄)-Alkyl, (C₃-C₈)-Cycloalkyl, (C₆-C₁₄)-Aryl, Phenyl, Naphthyl, Fluorenyl, (C₂-C₁₃)-Heteroaryl, wobei die Zahl der Heteroatome, ausgewählt aus der Gruppe N, O, S, 1-2 betragen kann, stehen und wobei
die vorgenannten Reste R unabhängig voneinander ein- oder mehrfach substituiert sein können und die Substituenten aus der Gruppe Wasserstoff, (C₁-C₂₀)-Alkyl, (C₂-C₂₀)-Alkenyl, (C₁-C₁₀)-Haloalkyl, (C₃-C₈)-Cycloalkyl, (C₂-C₉)-Heteroalkyl, (C₆-C₁₀)-Aryl, Phenyl, Naphthyl, Fluorenyl, (C₂-C₆)-Heteroaryl, wobei die Zahl der Heteroatome, ausgewählt aus der Gruppe N, O, S, 1-4 betragen kann, (C₁-C₁₀)-Alkoxy, (C₁-C₉)-Trihalomethylalkyl, Halogeno, Hydroxy, substituierte Amino der Formen NH-Alkyl-(C₁-C₈), NH-Aryl-(C₅-C₆), N(Alkyl-(C₁-C₈))₂, N(Aryl-(C₅-C₆))₂, N-Alkyl-(C₁-C₈)⁺₃, N-Aryl-(C₅-C₆)⁺₃, NH-CO-Alkyl-(C₁-C₈), NH-CO-Aryl-(C₅-C₆), Carboxylato der Formen COOH und COOQ wobei Q entweder ein einwertiges Kation oder (C₁-C₈)-Alkyl darstellt, (C₁-C₆)-Acyloxy, Sulfinato, Sulfonato der Formen SO₃H und SO₃Q, wobei Q entweder ein einwertiges Kation, (C₁-C₈)-Alkyl oder C₆-Aryl darstellt, Tri-(C₁-C₆)-Alkylsilyl ausgewählt sind, wobei auch zwei Reste R miteinander verbrückt sein können, wobei ein Zyklus gebildet wird, der gegebenenfalls mit linearen oder verzweigten Resten ausgewählt aus der Gruppe (C₁-C₁₀)-Alkyl, C₆-Aryl, Benzyl, (C₁-C₁₀)-Alkoxy, Hydroxy und Benzyloxy, substituiert ist und
X ein Wasserstoff, einen linearen oder verzweigten (C₁-C₁₀)-Alkyl, einen C₆-Aryl oder einen C(O)Y Rest darstellt, wobei Y für einen linearen oder verzweigten (C₁-C₁₀)-Alkyl-, C₆-Aryl- oder Benzylrest stehen kann und
P ein dreiwertiger Phosphor ist.

2. Verbindungen nach Anspruch 1, **dadurch gekennzeichnet, dass** die Reste R für 1-Methylethyl, tert-Butyl, Methyl, Ethyl, Cyclohexyl, Cyclopentyl, Phenyl, 2-Methyl-phenyl, 3,5-Dimethyl-phenyl, 4-Methyl-phenyl, 4-Methoxy-phenyl, 3,5-Bis-(trifluormethyl)-phenyl, 4-Trifluormethyl-phenyl, 3,5-Dimethyl-4-Methoxyphenyl, 4-Phenoxyl, 4-Dialkyamino, 2-Alkylphenyl, 3-Alkylphenyl, 4-Alkylphenyl, 2,6-Dialkylphenyl, 3,5-Dialkylphenyl, 3,4,5-Trialkylphenyl, 2-Alkoxyphenyl, 3-Alkoxyphenyl, 4-Alkoxyphenyl, 2,6-Dialkoxylphenyl, 3,5-Dialkoxyphenyl, 3,4,5-Triialkoxyphenyl, 3,5-Dialkyl-4-Alkoxyphenyl, 3,5-Dialkyl-4-dialkylaminophenyl, 4-Dialkylamino, 3,5-Trifluormethyl, 4-Trifluormethyl stehen.

3. Verbindungen nach Anspruch 1, **dadurch gekennzeichnet, dass** die Reste R für Phenyl, 2-Methyl-phenyl, 3,5-Dimethyl-phenyl, 4-Methyl-phenyl, 4-Methoxy-phenyl, 3,5-Bis-(trifluormethyl)-phenyl, 4-Trifluormethyl-phenyl, 3,5-Dimethyl-4-Methoxy-phenyl, Cyclohexyl und X für Wasserstoff, Methyl, Methoxymethyl, Ethyl oder Acetyl stehen.

4. Verbindungen nach einem der Ansprüche 1 bis 3, **dadurch gekennzeichnet, dass** die Verbindungen der Formel (I) enantiomerenangereichert sind.

5. Verbindungen nach Anspruch 4, **dadurch gekennzeichnet, dass** die Enantiomerenanreicherung 90 % übersteigt.

6. Komplexverbindungen erhältlich durch Zusammengeben mindestens eines Übergangsmetallsalzes oder eines Übergangsmetallvorkomplexes mit bidentaten Organophosphorliganden der Formel (I).

7. Komplexverbindungen der allgemeinen Formel (II)
[MₓP_{y}L_{z}S_{q}]Aᵣ (II)
wobei in der Formel (II)
M ein Übergangsmetallzentrum
L gleiche oder verschiedene koordinierende organische oder anorganische Liganden,
S koordinierende Lösungsmittelmoleküle und
A Äquivalente aus nicht koordinierenden Anionen repräsentiert,
wobei x 1 oder 2 ist, y eine ganzen Zahlen größer oder gleich 1 ist, z, q und r ganze Zahlen größer oder gleich 0 sind, wobei die Summe y + z + q durch die an den Metallzentren zur Verfügung stehenden Koordinationszentren nach oben begrenzt ist, wobei aber nicht alle Koordinationsstellen besetzt sein müssen,
**dadurch gekennzeichnet, dass**
P für erfindungsgemäße bidentate Organophosphorliganden der Formel (I) steht.

8. Komplexverbindung gemäß Anspruch 6 oder 7 **dadurch gekennzeichnet, dass** die Komplexverbindung ein Metall ausgewählt aus der Gruppe Ru, Co, Rh, lr, Ni, Pd, Pt, Cu enthält.

9. Verwendung einer Komplexverbindung nach einem der Ansprüche 6 bis 8 als Katalysator für asymmetrische Reaktionen oder Polymerisationen.

10. Verwendung einer Komplexverbindung nach einem der Ansprüche 6 bis 8 als Katalysator für asymmetrische Hydrierung, Hydroformylierung, Umlagerung, allylischen Alkylierung, Cyclopropanierung, Hydrosilylierung, Hydridübertragungsreaktionen, Hydroborierungen, Hydrocyanierungen, Hydrocarboxylierungen, Aldol Reaktionen oder Heck-Reaktionen.

11. Verwendung einer Komplexverbindung nach einem der Ansprüche 6 bis 8 als Katalysator für die asymmetrische Hydrierung und/oder Hydroformylierung.

## Claims

1. Diphosphine of the formula (I), 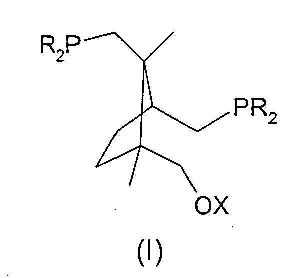 where the carbon skeleton of the compounds of the formula (I) can bear one or more linear, branched or cyclic (C₁-C₂₀)-alkyl substituents and the individual radicals
R are each, independently of one another, a radical selected from the group consisting of (C₁-C₂₄) -alkyl, (C₃-C₈) -cycloalkyl, (C₆-C₁₄)-aryl , phenyl, naphthyl, fluorenyl, (C₂-C₁₃)-heteroaryl, with the number of heteroatoms selected from the group consisting of N, O and S being able to be 1-2, and the abovementioned radicals R can each be, independently of one another, monosubstituted or polysubstituted and the substituents are selected from the group consisting of hydrogen, (C₁-C₂₀)-alkyl, (C₂-C₂₀)-alkenyl, (C₁-C₁₀) -haloalkyl, (C₃-C₈)-cycloalkyl, (C₂-C₉)-heteroalkyl, (C₆-C₁₀)-aryl, phenyl, naphthyl, fluorenyl, (C₂-C₆) -heteroaryl, with the number of heteroatoms selected from the group consisting of N, O and S being able to be 1-4, (C₁-C₁₀) -alkoxy, (C₁-C₉)-trihalomethylalkyl, halo, hydroxy, substituted amino of the formulae NH-alkyl- (C₁-C₈) , NH-aryl- (C₅-C₆) , N(alkyl- (C₁-C₈))₂, N (aryl- (C₅-C₆))₂, N-alkyl-(C₁-C₈) ⁺₃, N-aryl-(C₅-C₆)⁺₃, NH-CO-alkyl-(C₁-C₈), NH-CO-aryl-(C₅-C₆), carboxylato of the formulae COOH and COOQ, where Q is either a monovalent cation or (C₁-C₈)-alkyl, (C₁-C₆)-acyloxy, sulfinato, sulfonato of the formulae SO₃H and SO₃Q, where Q is either a monovalent cation, (C₁-C₈) -alkyl or C₆-aryl, tri- (C₁-C₆)-alkylsilyl, tri- (C₁-C₆) -alkylsilyl with two radicals R also being able to be joined to one another to form a ring which may be substituted by linear or branched radicals selected from the group consisting of (C₁-C₁₀) -alkyl, C₆-aryl, benzyl, (C₁-C₁₀)-alkoxy, hydroxy and benzyloxy, and
X is hydrogen, linear or branched (C₁-C₁₀)-alkyl, C₆-aryl or a C(O)Y radical, where Y can be a linear or branched (C₁-C₁₀) -alkyl, C₆-aryl or benzyl radical, and
P is a trivalent phosphorus.

2. Compound according to Claim 1, **characterized in that** the radicals R are 1-methylethyl, tert-butyl, methyl, ethyl, cyclohexyl, cyclopentyl, phenyl, 2-methylphenyl, 3,5-dimethylphenyl, 4-methylphenyl, 4-methoxyphenyl, 3,5-bis (trifluoromethyl) phenyl, 4-trifluoromethylphenyl, 3,5-dimethyl-4-methoxyphenyl, 4-phenoxyl, 4-dialkyamino, 2-alkylphenyl, 3-alkylphenyl, 4-alkylphenyl, 2,6-dialkylphenyl, 3,5-dialkylphenyl, 3,4,5-trialkylphenyl, 2-alkoxyphenyl, 3-alkoxyphenyl, 4-alkoxyphenyl, 2,6-dialkoxylphenyl, 3,5-dialkoxyphenyl, 3,4,5-trialkoxyphenyl, 3,5-dialkyl-4-alkoxyphenyl, 3,5-dialkyl-4-dialkylaminophenyl, 4-dialkylamino, 3,5-trifluoromethyl, 4-trifluoromethyl.

3. Compound according to Claim 1, **characterized in that** the radicals R are phenyl, 2-methylphenyl, 3,5-dimethylphenyl, 4-methylphenyl, 4-methoxyphenyl, 3,5-bis(trifluoromethyl)phenyl, 4-trifluoromethylphenyl, 3,5-dimethyl-4-methoxyphenyl, cyclohexyl, and X is hydrogen, methyl, methoxymethyl, ethyl or acetyl.

4. Compound according to any one of Claims 1 to 3, **characterized in that** the compound of the formula (I) is enantiomerically enriched.

5. Compound according to Claim 4, **characterized in that** the enantiomeric enrichment exceeds 90%.

6. Complex obtainable by combining at least one transition metal salt or a transition metal precomplex with bidentate organophosphorus ligands of the formula (I).

7. Complex of the general formula (II)
[MₓP_{y}L_{z}S_{q}] Aᵣ (II)
where, in the formula (II)
M represents a transition metal center,
L represents identical or different coordinating organic or inorganic ligands,
S represents coordinating solvent molecules and
A represents equivalents of noncoordinating anions,
where x is 1 or 2, y is an integer greater than or equal to 1, z, q and r are integers greater than or equal to 0, with the upper limit to the sum y + z + q being imposed by the coordination sites available on the metal centers but not all coordination sites having to be occupied,
**characterized in that**
P is a bidentate organophosphorus ligand of the formula (I) according to the invention.

8. Complex according to Claim 6 or 7, **characterized in that** the complex contains a metal selected from the group consisting of Ru, Co, Rh, Ir, Ni, Pd, Pt, Cu.

9. Use of a complex according to any one of Claims 6 to 8 as catalyst for asymmetric reactions or polymerizations.

10. Use of a complex according to any one of Claims 6 to 8 as catalyst for asymmetric hydrogenation, hydroformylation, rearrangement, allylic alkylation, cyclopropanation, hydrosilylation, hydride transfer reactions, hydroborations, hydrocyanations, hydrocarboxylations, aldol reactions or Heck reactions.

11. Use of a complex according to any one of Claims 6 to 8 as catalyst for asymmetric hydrogenation and/or hydroformylation.

## Revendications

1. Diphosphines de formule (I) 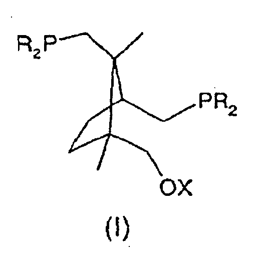 dans laquelle le squelette carboné des composés de formule (I) peut porter un ou plusieurs substituants alkyle en C₁-C₂₀ à chaîne droite ou ramifiée, ou cycliques, et où les différents radicaux ont les significations suivantes :
les radicaux R représentent, chacun indépendamment de l'autre, un radical choisi dans l'ensemble consistant en les groupes alkyle en C₁-C₂₄, cycloalkyle en C₃-C₈, aryle en C₆-C₁₄, phényle, naphtyle, fluorényle, hétéroaryle en C₂-C₁₃, le nombre des hétéroatomes, choisis parmi N, O, S, pouvant être de 1-2, et où
les radicaux R mentionnés ci-dessus peuvent, indépendamment l'un de l'autre, être une ou plusieurs fois substitués, et les substituants peuvent être choisis dans l'ensemble consistant en l'atome d'hydrogène, les groupes alkyle en C₁-C₂₀, alcényle en C₂-C₂₀, halogènalkyle en C₁-C₁₀, cycloalkyle en C₃-C₈, hétéroalkyle en C₂-C₉, aryle en C₆-C₁₀, phényle, naphtyle, fluorényle, hétéroaryle en C₂-C₆, le nombre des hétéroatomes, choisis parmi N, O, S pouvant être de 1-4, alcoxy en C₁-C₁₀, trihalogénométhyl (alkyle en C₁-C₉), halogéno, hydroxy, amino substitué ayant les formes NH-(alkyle en C₁-C₈) , NH- (aryle en C₅-C₆), N(alkyle en C₁-C₈)₂, N(aryle en C₅-C₆)₂, N- (alkyle en C₁-C₈)⁺₃, N-(aryle en C₅-C₆)⁺₃, NH-CO- (alkyle en C₁-C₈), NH-CO- (aryle en C₅-C₆), carboxylato ayant les formes COOH et COOQ, où Q représente un cation monovalent ou un groupe alkyle en C₁-C₈, acyloxy en C₁-C₆, sulfinato, sulfonato ayant les formes SO₃H et SO₃Q, où Q est un cation monovalent ou un groupe alkyle en C₁-C₈ ou aryle en C₆, tri (alkyle en C₁-C₆)silyle, auquel cas deux radicaux R peuvent aussi être pontés l'un à l'autre, en formant un cycle, qui éventuellement est substitué par des radicaux à chaîne droite ou ramifiée qui sont choisis dans l'ensemble consistant en les groupes alkyle en C₁-C₁₀, aryle en C₆, benzyle, alcoxy en C₁-C₁₀, hydroxy et benzyloxy, et
X est un atome d'hydrogène, un radical alkyle en C₁-C₁₀ à chaîne droite ou ramifiée, aryle en C₆ ou C (O) Y, Y pouvant être un radical alkyle en C₁-C₁₀ à chaîne droite ou ramifiée, aryle en C₆ ou benzyle, et
P est un phosphore trivalent.

2. Composés selon la revendication 1, **caractérisés en ce que** les radicaux R désignent les groupes 1-méthyl-éthyle, tert-butyle, méthyle, éthyle, cyclohexyle, cyclopentyle, phényle, 2-méthyl-phényle, 3,5-diméthyl-phényle, 4-méthyl-phényle, 4-méthoxy-phényle, 3,5-bis(trifluorométhyl)-phényle, 4-trifluorométhyl-phényle, 3,5-diméthyl-4-méthoxy-phényle, 4-phénoxyle, 4-dialkylamino, 2-alkyphényle, 3-alkylphényle, 4-alkylphényle, 2,6-dialkylphényle, 3,5-dialkylphényle, 3,4,5-dialkylphényle, 2-alcoxyphényle, 3-alcoxyphényle, 4-alcoxyphényle, 2,6-dialcoxyphényle, 3,5-dialcoxyphényle, 3,4,5-trialcoxyphényle, 3,5-dialkyl-4-alcoxyphényle, 3,5-dialkyl-4-dialkylaminophényle, 4-dialkylamino, 3,5-trifluorométhyle, 4-trifluorométhyle.

3. Composés selon la revendication 1, **caractérisés en ce que** les radicaux R représentent les groupes phényle, 2-méthyl-phényle, 3,5-diméthyl-phényle, 4-méthyl-phényle, 4-méthoxy-phényle, 3,5-bis(trifluorométhyl)-phényle, 4-trifluorométhyl-phényle, 3,5-diméthyl-4-méthoxy-phényle, cyclohexyle et X est un atome d'hydrogène ou un groupe méthyle, méthoxyméthyle, éthyle ou acétyle.

4. Composés selon l'une des revendications 1 à 3, **caractérisés en ce que** les composés de formule (I) sont enrichis en énantiomères.

5. Composés selon la revendication 4, **caractérisés en ce que** l'enrichissement en énantiomères est supérieur à 90 %.

6. Composés complexes pouvant être obtenus par réunion d'un sel d'un métal de transition ou d'un précomplexe d'un métal de transition avec des ligands organophosphorés bidentés de formule (I).

7. Composés complexes de formule générale (II)
[MₓP_{y}L_{z}S_{q}]Aᵣ (II)
dans lesquels, dans la formule (II) :
M est un centre métal de transition,
L représente des ligands organiques ou inorganiques coordinants, identiques ou différents
S représente des molécules d'un solvant coordinant, et
A représente des équivalents d'anions non coordinants, x valant 1 ou 2, y étant un nombre entier supérieur ou égal à 1, z, q et r étant des nombres entiers supérieurs ou égaux à 0, la somme y + z + q étant limitée vers le haut, par les centres de coordination disponibles au niveau des centres métalliques, mais tous les sites de coordination ne devant pas être occupés,
**caractérisés en ce que**
P représente des ligands organophosphorés bidentés selon l'invention de formule (I).

8. Composés complexes selon la revendication 6 ou 7, **caractérisés en ce que** le composé complexe contient un métal choisi dans l'ensemble consistant en Ru, Co, Rh, Ir, Ni, Pd, Pt, Cu.

9. Utilisation d'un composé complexe selon l'une des revendications 6 à 8 en tant que catalyseur pour des réactions ou des polymérisations asymétriques.

10. Utilisation d'un composé complexe selon l'une des revendications 6 à 8 en tant que catalyseur pour des réactions asymétriques d'hydrogénation, d'hydroformylation, de transposition, d'alkylation allylique, de cyclopropanation, d'hydrosilylation, de transfert d'hydrure, d'hydroboration, d'hydrocyanation, d'hydrocarboxylation, de réactions aldol ou de réactions de Heck.

11. Utilisation d'un composé complexe selon l'une des revendications 6 à 8 en tant que catalyseur pour une hydrogénation et/ou une hydroformylation asymétriques.
